# EUROPEAN PATENT SPECIFICATION

(11) **EP 1 727 814 B1**
(45) Date of publication and mention of the grant of the patent: **30.04.2008**
(21) Application number: 05716809.8
(22) Date of filing: 25.02.2005
(51) Int. Cl.: C07D 413/04, C07D 473/18, C07D 473/34, A61K 31/513, A61K 31/52, A61K 31/522, A61P 31/12

(54) **NUCLEOSIDE ANALOGUES WITH ANTIVIRAL ACTIVITY**
NUKLEOSIDANALOGA MIT ANTIVIRALER WIRKUNG
ANALOGUES DE NUCLEOSIDES A ACTIVITE ANTIVIRALE

(30) Priority: 26.02.2004 IT RM2004 000
(43) Date of publication of application: 06.12.2006
(73) Proprietor: Universita' Degli Studi Di Catania, 95124 Catania (IT); Università Degli Studi Di Messina, 98122 Messina (IT)
(72) Inventor: CHIACCHIO, Ugo, I-95024 ACIREALE (IT); CORSARO, Antonino, I-95027 S. GREGORIO DI CATANIA (IT); IANNAZZO, Daniela, I-98021 ALI' TERME (IT); MACCHI, Beatrice, I-00141 ROMA (IT); MASTINO, Antonio, I-00141 ROMA (IT); PIPERNO, Anna, I-89018 VILLA SAN GIOVANNI (IT); RESCIFINA, Antonio, I-95022 ACI CATENA (IT); ROMEO, Giovanni, I-98168 MESSINA (IT); ROMEO, Roberto, I-98168 MESSINA (IT)
(74) Representative: Gervasi, Gemma
(86) International application number: PCT/EP2005/050818
(87) International publication number: WO 2005/082896

(56) References cited:
- CHIACCHIO, UGO ET AL: "Enantioselective Syntheses and Cytotoxicity of N,O-Nucleosides" JOURNAL OF MEDICINAL CHEMISTRY , 46(17), 3696-3702 CODEN: JMCMAR; ISSN: 0022-2623, 2003, XP002339663 cited in the application
- CHIACCHIO, U. ET AL.: "Diastereoselective and enantioselective synthesis of 4'-aza analogues of 2',3'-dideoxynucleosides" TETRAHEDRON: ASYMMETRY, vol. 11, 2000, pages 2045-2048, XP004205758 cited in the application

## Description

### FIELD OF THE INVENTION

The present invention relates to the field of nucleoside compounds, and in particular to the nucleoside analogues with antiviral activity of formula (I) hereinafter reported, to the processes for their preparation and to their use.

### STATE OF THE ART

The biological activity of nucleoside analogues (ddNs) which exhibit antiviral activity and antitumour properties is closely correlated to their conversion, through cellular enzymes, into corresponding 5'-mono-, di- and triphosphates, which interact with the HIV-associated reverse transcriptase (Richman, D.D. Nature, 2001, 410, 995-1001) or interfere with cell growth, slowing down cell cycle progression (Macchi B. et al. Journal of General Virology, 1997, 78, 1007-1016).

In many cases, amongst the three successive phosphorylation steps, the first step is the one that limits speed, while further conversions to di- and tri-phosphates are catalysed by less specific kinases (Perigaud C. et al. Advances in Antiviral Drug Design De Clerq, E. Ed., 1995, vol. 2, pp 167-172).

It is known that various nucleoside inhibitors, including those currently used in AIDS therapy, are antiproliferative and are capable of interacting with nucleic acids. Recently, it has been shown that zidovudine (AZT) and didanosine (DDI) cause dose-dependent inhibition of the proliferative response, in human peripheral blood mononuclear cells stimulated by interleukin 2, acting as mitogens, with down-regulation of the IL-2a receptor and of the IL-2 receptor, through gene activation.

Inhibition of the cell cycle could be explained with the fact that, being a nucleoside analogue of thymidine, AZT acts as a substrate not only for reverse transcriptase, but also, although much less efficiently, for cellular DNA polymerase, inhibiting lengthening of the DNA chain in replication. Therefore, cell-nucleoside inhibitor interaction causes damage to the cell DNA, which could lead to induction of cell death by apoptosis. In fact, it has been shown that cell lines from patients with AIDS-correlated Burkitt's Lymphoma (BL), resistant to conventional chemotherapy, treated with AZT *in vitro,* were sensitive to apoptosis, very probably correlated to an increase in the levels of AZT monophosphate (Sussman, H.E. et al. in Mutation Res. 1999, 429, 249-259).

Various strategies have been hypothesised to overcome the problem of the initial selective step of phosphorylation, and of genetic damage caused by some antiretroviral agents currently in use.

One possibility to improve the efficiency of ddNs might be to bypass the phosphorylation step; unfortunately, due to their polar nature, nucleotides are unable to cross the cell membrane efficaciously. Moreover, ddNs are easily dephosphorylated in extracellular fluids and on the surfaces of cells by non-specific phosphohydrolases (Cooperwood, J. S. et al. Recent Advances in Nucleosides: Chemistry and Chemotherapy 2002, 91-147).

The use of prodrugs (pronucleotides), which incorporate protection groups of the enzyme-labile phosphate group, has recently emerged, and a large number of prodrug derivatives of nucleoside monophosphates have been prepared.

A logical approach is to produce phosphate analogues in which the phosphoric unit is substituted by isosteric and isoelectronic phosphonates. These analogues, enzymatically and chemically stable, mime the nucleoside monophosphates and are capable of overcoming the instability of the nucleotides towards phosphodiesterases and of increasing their cell uptake, avoiding the initial step of enzymatic phosphorylation.

In this context, synthesis of a series of ddNs in which the furanose ring was substituted by an N,O-heterocyclic ring was developed (Chiacchio U. et al. Tetrahedron Asym. 2000, 11, 2045; Merino P. Curr. Med. Chem., 2002, 1, 389).

Isoxazolidinyl nucleosides of general formula **1** constitute a new class of compounds which exhibited interesting biological activities (Chiacchio, U. et al. J. Med Chem., 2003, 46, 3696-3702) 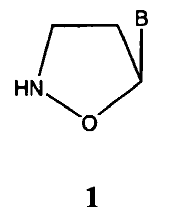 wherein B is a nucleic acid base.

### SUMMARY OF THE INVENTION

The Applicant has now prepared new phosphonated isoxazolidinyl nucleosides of general formula (I) hereinafter reported which, as a function of the specificity of the nucleobase, have shown to possess *in vitro* low cytotoxic activity of the classic type, moderate proapoptotic activity towards human lymphoid or monocytoid cells and high activity towards experimental models of viral infection. They can therefore be used as antiviral agents.

Subject of the invention are therefore the nucleoside analogues of general formula (I) 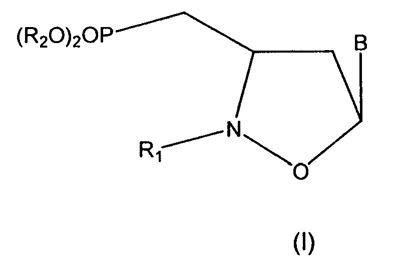 wherein:
B is **a group selected from: thymine, n-acetylcytosine, uracil, 5-fluorouracil, cytosine, guanine, adenine**,
R₁ is selected from the group consisting of H, C1-C4 alkyl groups, aryl groups, and glycoside units,
R₂ is selected from the group consisting of H, C1-C4 alkyl groups, aryl groups, and **protective groups easily hydrolyzable *in vivo***, and their pharmaceutically acceptable salts.

Further subject of the invention are the preparation processes of the aforesaid compounds of formula (I), and their use for the preparation of pharmaceutical compositions for the treatment of viral pathologies, and said pharmaceutical compositions.

Features and advantages of the invention will be illustrated in detail in the following description.

### DETAILED DESCRIPTION OF THE INVENTION

Within the scope of the present invention the symbol "Et" indicates the Ethyl group, whereas by the expression "nucleic acid base", unless otherwise specified, it is meant one of the five bases which, together with ribose and phosphate groups, constitute the nucleic acids, i.e. guanine, adenine, uracil, thymine and cytosine.

The group B according to the invention is in fact selected from the nucleic acid bases and their derivatives, such as fluorouracil and N-acetylcytosine.

The term "C1-C4 alkyl groups", as used in the present invention, should be meant, for example, as methyl, ethyl, propyl and butyl groups, while the term "aryl groups" should be meant for example as the benzyl group, optionally substituted, and heteroaromatic groups, in particular pyridyl and pyrrole groups.

In the compounds of formula (I) according to the invention the group R₁ is preferable chosen from between H and methyl; when R₁ is a glycoside unit, it is preferably ribosyl acetonide.

The group R₂ is preferable ethyl.

As protective groups easily hydrolyzable *in vivo,* **are intended** isopropyloxymethylcarbonyl (POC) group and the pivaloyloxymethyl (POM) group.

The expression "nucleosides of general formula (I)", unless otherwise specified, is intended to include the racemic mixtures, individual isolated enantiomers and the individual diastereomers α and β.

The compounds of formula (I) according to the invention can be prepared with a process comprising the following steps:
i) hydrolysis of the diethyl acetal of phosphonoacetaldehyde of formula (II) to obtain the phosphonoacetaldehyde of formula (III) 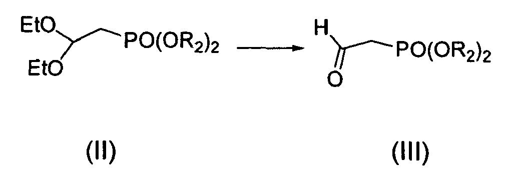 wherein R₂ is as defined above;
(IV) 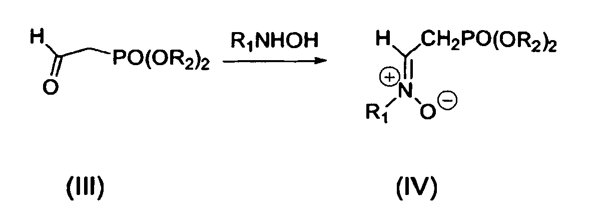 wherein R₁ and R₂ are as defined above;
iii) cycloaddition of the nitrone of formula (IV) coming from step ii) with vinylacetate to obtain the mixture of isoxazolidine epimers of formula (V) and (VI) 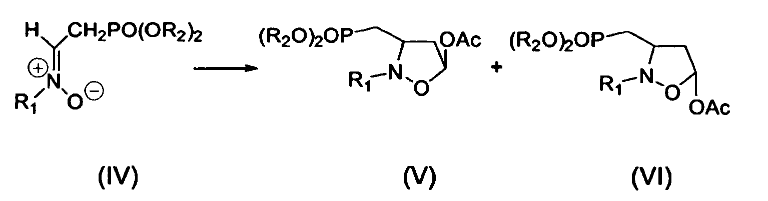 wherein R₁ and R₂ are defined as above;
iv) reaction of the epimeric mixture of the compounds of formula (V) and (VI) obtained from step iii) with a silylated nucleic acid base or a derivative thereof, to obtain the nucleoside analogue of formula (I) 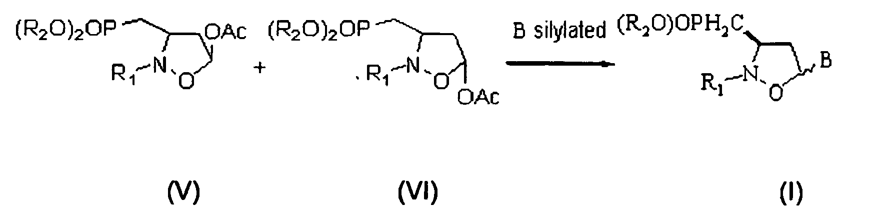
wherein R₁ and R₂ are defined as above, B is a nucleic acid base or a derivative thereof, and the bond represented by the broken line indicates that the compound of formula (I) is obtained in the form of mixtures of α- and β- nucleosides.

The starting compound of formula (II) in the present process wherein R₂ is ethyl, that is, the diethyl acetal of the diethylphosphonoacetaldehyde, is a commercially available product, while the other compounds of formula (II) wherein R₂ is different from ethyl, can be prepared starting from this commercially available product by transesterification according to procedures commonly used and known to those skilled in the art.

Step i) of the present process is for example carried out by treating the diacetal of formula (II) with an ion exchange resin, for example with Amberlist^{®}-15, in acetone at room temperature.

Step ii) which implies the formation of nitrone (IV) is carried out by reaction of the diethylphosphonoacetaldehyde (III) with the suitable hydroxylamine according to the product to be obtained in an organic solvent, for example in dichloromethane.

Cycloaddition in step iii) of the present process produces an epimeric mixture wherein the ratio between the epimer of formula (V) and the epimer of formula (VI) is for example 2.5:1.

The crude mixture of the two epimers of formula (V) and (VI) obtained from step iii) can be purified to obtain the two separated epimers, for example by flash chromatography using chloroform as eluent, which are then subjected separately to the subsequent reaction step iv) with the silylated bases.

Alternatively, the crude mixture of the two epimers of formula (V) and (VI) can be subjected as is to step iv), obtaining in any case the final product of formula (I) in the form of epimeric mixture, wherein the ratio between the epimers α and β does not change with respect to the reaction carried out on the two separate epimers (V) and (VI).

According to a preferred embodiment of the present process, the reaction in step iv) between the silylated base and the two epimers of formula (V) and (VI) separated or in the form of epimeric mixture, obtained from step iii), is carried out by condensation according to the procedure described by Vorbrüggen H. et al. in Chem. Ber. 1981, 114, 12340. According to this procedure, the condensation reaction is carried out in acetonitrile at a temperature ranging from 20 to 70°C, in the presence of 0.1-0.4 equivalents of trimethylsilyl trifluoromethanesulfonate, hereinafter indicated with the abbreviation "TMSOTf", as catalyst.

Alternatively, the nucleosidation reaction in step iv) can be carried out using stannic chloride as catalyst in amounts ranging from 0.1 to 0.4 equivalents, at a temperature ranging from 20 to 70°C in an anhydrous organic solvent selected in the group consisting of acetonitrile, dichloromethane, dichloroethane, tetrahydrofurane and dimethylformamide.

The nucleic acid bases or their derivatives are introduced into the present nucleoside analogues in the form of derivatives silylated at the oxygen or nitrogen atoms, that can be easily prepared according to procedures commonly used and known to those skilled in the art, by treating nucleic acid bases with N,O-bis-trimethylsilylacetamide or hexamethyldisilazan.

If necessary, the mixture of α- and β-nucleosides obtained from step iv) of the present process can be purified to obtain the two separated epimers, for example through silica gel column chromatography and flash chromatography using as eluent a mixture chosen from chloroform-methanol 98:2 or ethylacetate-cyclohexane 7:3.

The ratio between the epimers α and β of the nucleoside analogues of formula (I) obtained with the present process, ranges from 1:99 to 30:70.

Alternatively, the present compounds of general formula (I) can be prepared by reaction of the nitrones (IV) with the corresponding vinyl nucleobases (Dalpozzo A., et al. Synthesis, 2002, 2, 172). This cycloaddition reaction is preferably carried out in toluene at a temperature ranging from 80 to 90°C for 24 hours.

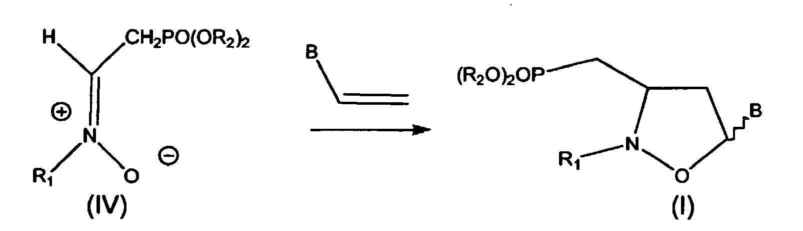

The nitrones of formula (IV) can be prepared as described in the process indicated above in steps i) and ii).

The nucleosides of general formula (I), subject of the present invention, are accessible through a further synthetic route that starts from the nitrone of formula (VII) (lannazzo D. et al., Tetrahedron, 2002, 58, 581-587).

The nitrone of general formula (VII) wherein R₁ is as defined above and R₃ is a C1-C4 alkyl group, made to react with vinylacetate in the conditions indicated previously, provides the epimeric isoxazolidines of formula (VH) and (IX) which are directly coupled with the nucleobases indicated according to Vorbruggen's procedure.

Subsequent treatment with tetrabutyl ammonium fluoride and then with thionyl chloride leads to the derivates of formula (X) (relative ratio 1:1) which are converted into the nucleosides of general formula (I) wherein R₂ is Et, by means of Arbuzov reaction with triethyl phosphite.

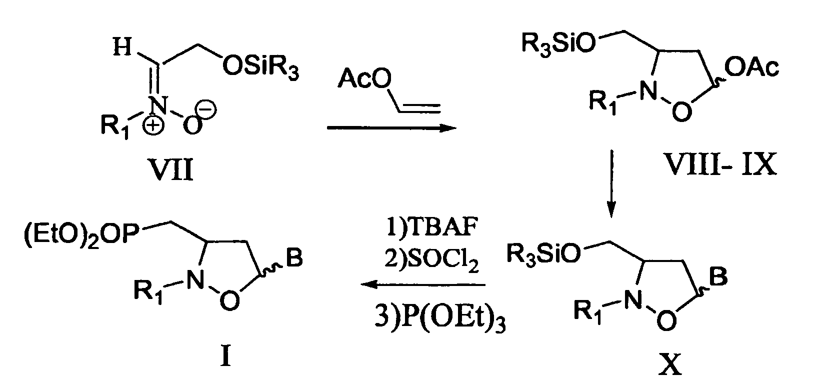

The nucleosides of general formula (I), wherein R₁ is H or a glucoside unit, are obtained in enantiomerically pure form exploiting chiral induction induced by a sugar unit introduced on the nitrogen atom, according to the scheme described below for the nucleosides of formula (I) wherein R₂ is Et. According to this procedure, the 2,3-isopropylidene-D-ribose of formula (XI) is protected at the primary hydroxyl, by treatment with t-butyl-diphenyl-silyl chloride, and then transformed, by reaction with hydroxylamine hydrochloride, into the ribosyl oxime of formula (XIII).

Further treatment with diethylphosphonoacetaldehyde of formula (III) and vinylacetate provides, through the transient, non-isolated nitrone of formula (XIV), a mixture of the two epimeric isoxazolidines of formula (XV) and XVI) in enantiomerically pure form (relative ratio 75:25; overall yield 70%).

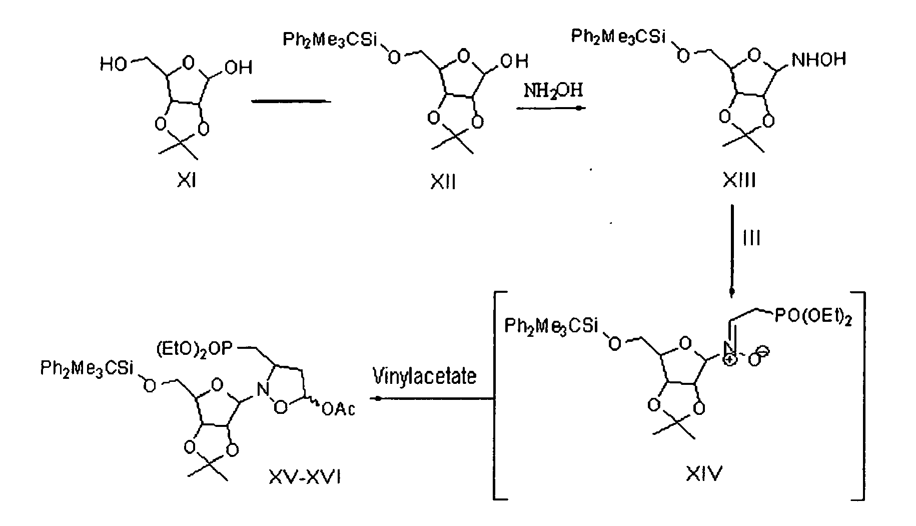

The crude mixture of the two epimers of formula (XV) and (XVI) can be purified to obtain the two separated epimers, for example by flash chromatography using chloroform as eluent, which are then subjected separately to the subsequent reaction step with the silylated bases.

Alternatively the crude mixture of the two epimers of formula (XV) and (XVI) can be subjected as such to the nucleosidation step, thus obtaining the epimeric mixture of nucleosides of formula (XVII) and (XVIII), enantiomerically pure, wherein the ratio between the epimers α and β (1:4) does not change with respect to the reaction carried out on the two separated epimers of formula (XV) and (XVI).

The synthetic scheme is thus completed by removing the silylated protective group by treatment with TBAF to obtain the two nucleosides of formula (I) wherein R₂ is Et and R₁ is a glycoside unit in enantiomerically pure form α and β.

Moreover, the glycoside unit present on the nitrogen atom can be eliminated directly by treatment with an ethanolic solution with 1.5% of HCl or using ion exchange resins: this yields nucleosides of formula (I) wherein R₁ is H in enantiomerically pure form α and β.

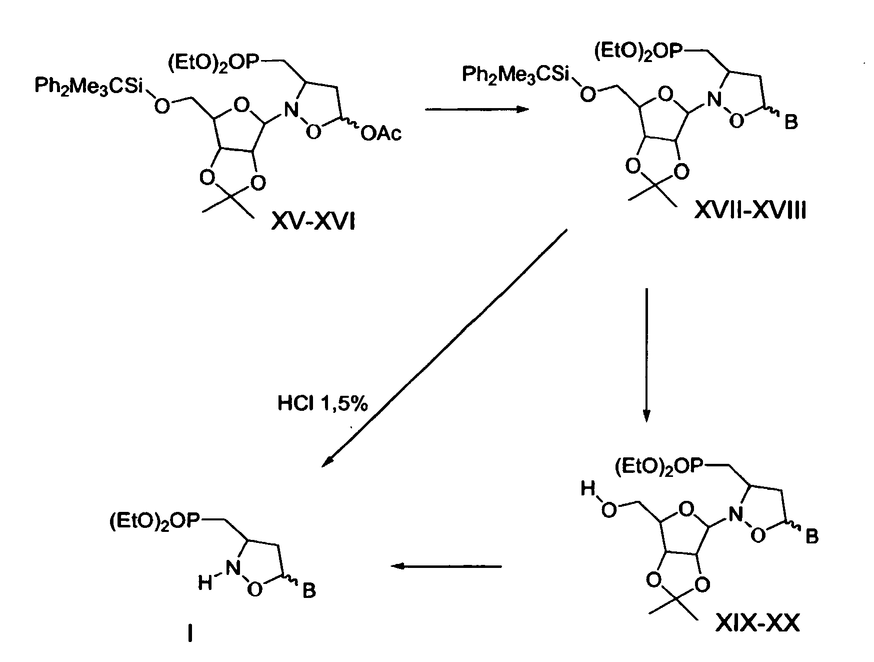

Alternatively, the products of formula (I) wherein R₁ is H or a glycoside unit, in the two separated enantiomerically pure forms α and β, can be prepared through reaction of the nitrone of formula (XIV) with the corresponding vinyl nucleobases (Dalpozzo A., et al. Synthesis, 2002, 2, 172).

The present compounds of general formula (I), in free form or in the form of pharmaceutically acceptable salts, can be used for the preparation of pharmaceutical compositions following conventional preparation methods in the pharmacological field.

These pharmaceutical compositions can be formulated conventionally, and may comprise one or more pharmaceutically acceptable excipients and/or diluents known to those skilled in the art.

These compositions can be administered using any conventional administration method, for example parenterally, in the form of injectable solutions or suspensions, orally, topically, nasally, etc.

The formulations of these pharmaceutical compositions according to the invention include pills, capsules, tablets, solutions, dispersions, suspensions, collyriums, liposomal formulations, microspheres, nanospheres, creams and ointments, emulsions and aerosols, and can also be prepared to produce controlled or delayed release of the active ingredient.

The doses and administration methods vary as a function of the type and severity of the pathological condition to be treated.

Moreover, these pharmaceutical compositions can optionally comprise the present nucleoside analogues of formula (I) in combination with other active ingredients or adjuvants, chosen according to the pathological conditions to be treated.

The present pharmaceutical compositions comprising the compounds of the invention are suitable for the pharmacological treatment of viral pathological conditions, and in particular for the treatment of retrovirus, hepatic virus and herpes virus infections.

The present nucleoside analogues of general formula (I) have in fact shown to possess *in vitro* antiviral activity in some experimental infection models assayed by way of example, a detailed description of which is provided in the experimental section reported hereinafter.

Moreover, they have also shown to possess moderate proapoptotic activity with regard to human lymphoid or monocytoid cells as a function of the specificity of the nucleobase, proving in particular capable of strengthening the action of the death receptor Fas.

The following examples are provided by way of non-limiting illustrations of the present invention.

### EXAMPLE 1

### Preparation of the compound of formula (III) wherein R₂ is Et

10 mmol of diethylphosphonoacetaldehyde diethyl acetal (compound of formula (II) wherein R₂ is Et) and 0.4 g of Amberlist^{®}-15 were added to a solution of 40 ml of acetone containing 0.6 ml of water. The mixture was left under stirring for 24 hours at room temperature, filtered and then evaporated at reduced pressure.

The residue purified by flash chromatography using a chloroform methanol mixture 98:2 as eluent provided the pure aldehyde of formula (III) wherein R₂ is Et (yield = 90%).

### EXAMPLE 2

### Preparation of the compound of formula (IV) wherein R₂ is Et and R₁ is Me

10 mmol of product of formula (III) prepared as described above in Example 1 was made to react with 10 mmol of N-methyl hydroxylamine in 80 ml of dichloromethane for 5 hours. At the end of this time the mixture was taken to dryness, and the product of the title thus obtained was used as is in the subsequent steps.

### EXAMPLE 3

### Preparation of the epimeric mixture of the compounds of formula (V) and (VI) wherein R₂ is Et and R₁ is Me

10 mmol of nitrone (IV) as described in Example 2 above was made to react with 30 ml of vinylacetate for 12 hours at 50-55°C. The reaction mixture was then evaporated at reduced pressure and the residue chromatographed with a chloroform-methanol eluent mixture in the ratio 99:1. The first eluted product was the isoxazolidine of formula (V) wherein R₂ is Et and R₁ is Me, which was characterised by NMR spectra, M/s and elementary analysis (yield = 64%). The second eluted product was the isoxazolidine of formula (VI) wherein R₂ is Et and R₁ is Me with a yield of 26%.

### EXAMPLE 4

### Preparation of the compound of formula (I) wherein R₂ is Et, R₁ is Me and B is fluorouracil

N,O-bis(trimethylsilyl)acetamide (2.54 mmol) was added to a suspension of fluorouracil (0.618 mmol) in anhydrous acetonitrile (3 ml). A solution in anhydrous acetonitrile (3 ml) of the epimeric mixture of isoxazolidine of formula (V) and (VI) (0.517 mmol), prepared as described above in Example 3, was added to the clarified solution; subsequently trimethylsilyl triflate (0.18 mmol) was added. The reaction mixture was then left under stirring at 50°C for 6h and after cooling, the solution was neutralised using a solution of sodium bicarbonate 5% and vacuum concentrated. Dichloromethane (8 ml) was then added to the crude product and, after extraction, the organic phase was washed with water (2 x 10 ml), dried on sodium sulfate, and vacuum concentrated, to obtain the product of the title in the form of epimeric mixture. The mixture was then subjected to flash chromatography using as eluent the mixture cyclohexane/ethyl acetate 3:7, from which the product of the title with a ratio α/β 1.0 : 99.0 was obtained.

### EXAMPLE 5

### Preparation of the compound of formula (I) wherein R₂ is Et, R₁ is Me and B is fluorouracil

A solution of the vinyl base, vinylfluorouracil (1.5 eq) and of the nitrone of formula (IV) wherein R₁ is Me and R₂ is Et (1 eq) in anhydrous toluene was heated in a closed vial at 80°C under stirring for 24 hours. After vacuum removal of the solvent a crude product was obtained which was purified by flash chromatography to give a mixture of the nucleosides of formula (I) (ratio α/β 1.0:1.5; 40% global product), which were purified by flash chromatography (cyclohexane/ethyl acetate 3:7).

### EXAMPLE 6

### Preparation of the compound of formula (1) wherein R₂ is Et, R₁ is Me and B is fluorouracil

0.1 moles of D-mannitol were made to react with 0.22 moles of diphenyl tert-butylsilyl chloride and 0.44 moles of imidazole in 100 ml of anhydrous DMF. The mixture was stirred for 12 hours and subsequently the solvent was eliminated. The crude reaction product was treated with 3 equivalents of lead acetate in 100 ml of benzene. The reaction mixture was stirred for 3 hours and finally extracted with dichloromethane and water. The organic phases were dried on sodium sulfate, taken to dryness and purified by chromatography using ethyl ether/hexane 2:8 as eluent mixture.

The aldehyde obtained (0.1 moles) was made to react with 0.1 moles of methyl hydroxylamine in 50 ml of dichloromethane for 6 hours at room temperature. The reaction mixture was taken to dryness to obtain the desired nitrone of formula (VII) wherein R₁ is Me and R₃ is diphenyl-t-butyl with quantitative yield.

0.1 moles of silylated nitrone were made to react with 50 ml of vinylacetate at reflux for 12 hours. The alkene in excess was removed at reduced pressure and the crude reaction product was coupled with silylated bases using the procedure described previously. The crude reaction product column-purified using cyclohexane/acetate 7:3, produced the isoxazolidines of formula (VIII) and (IX) wherein R₁ is Me and R₃ is diphenyl-t-butyl with a respective yield of 65% and 32%.

A solution of the nucleosides of formula (VIII) and (IX) (0.1 moles) in anhydrous THE was treated with 10.5 ml of TBAF (0.11 moles) and left under stirring at room temperature for 1.5 hours. The solvent was then removed and the residue chromatographed with chloroform/methanol 95:5 to give the respective deprotected isoxazolidines.

The deprotected isoxazolidines were treated with 1 eq. of thionyl chloride in 50 ml of anhydrous chloroform, and the resulting solution stirred for 6 hours. The reaction mixture was evaporated and the residue treated with 1.3 eq. of triethylphosphite at 90°C for 3 hours, after flash chromatography gives the nucleoside of formula (I) of the title.

### EXAMPLE 8

### Preparation of the compounds of formula (XV) and (XVI)

A solution of (4S,5R)-5-[(1R)-2[[1-(ter-butyl)-1,1-diphenylsilyl]oxy]-1-hydroxyethyl]-2,2-dimethyl-1,3-dioxolane-4-carbaldehyde oxime of formula (XIII) prepared as described by Chiacchio U. et al. in J. Org. Chem. 199, 24, 9321-9327 (4.23 g, 9.87 mmol), vinylacetate (18.2 ml, 197.44 mmol) and diethyl phosphonoacetaldehyde of formula (III) (12.8 mmol; 50% solution in toluene) was heated to 60°C, in a closed vial, for 14 hours. The reaction mixture was evaporated and the residue was purified using a chromatographic column (cyclohexane/ethyl acetate 4:1) and subsequently by HPLC (*n*-hexane/isopropanol 9.5:4.5).

The first eluted product was the compound of formula (XV) (3S,5R)(cis) major compound. The fraction eluted subsequently gave the compound of formula (XVI) (3S, 5S) (trans) minor compound.

### EXAMPLE 9

### Preparation of (3'S,5'S)- and (3'S,5'R)-1-[2'-(5"-O-tert-Butyldiphenylsilyl-2",3"-O-isopropylidene-β-D-ribofuranosyl)-3'-diethoxyphosphonylmethylene-1',2'-isoxazolidinyl]-5-fluorouracil [compounds of formula (XVII) and (XVIII)]

A suspension of 5-fluorouracil (78 mg, 0.618 mmol) in anhydrous acetonitrile (3 ml) was treated with bis(trimethylsilyl)acetamide (0.62 ml, 2.54 mmol) for 15 min at reflux. The mixture of the isoxazolidines of formula (XV) and (XVI) prepared as described above in Example 8 (310 mg, 0.517 mmol) in anhydrous acetonitrile (3 ml), and subsequently trimethylsilyltriflate (174 mg, 0.78 mmol) were added to the resulting solution. The solution was left at reflux for 1 h. After having taken the temperature of the mixture to 0°C, the solution was neutralised by very carefully adding an aqueous solution of sodium bicarbonate 5%; finally, the solution was vacuum concentrated. After the addition of dichloromethane (8 ml), the organic phase was separated, washed with water (2 x 10 ml), dried on sodium sulphate, filtered and vacuum evaporated. The residue was purified by radial chromatography (cyclohexane/ethyl acetate 3:2) and subsequently by HPLC using as eluent an isopropanol/ethyl acetate mixture 35:65 (flux 3.5 ml/min). The product eluted first was the compound β (XVII). The subsequent elution gave the compound α (XVIII) (relative ratio β/α 75:25).

### EXAMPLE 10

### Preparation of (3'R,5'R)- and (3'R,5'S)-1-(3' diethoxyphosphonylmethylene-1'.2'-isoxazolidinyl)-5-fluorouracil [compound of formula (I) β and (I) α]

The compound of formula (XVII) or the compound of formula (XVIII) (100 mg, 0.15 mmol) was dissolved in a 1.5% solution of HCl in EtOH (2.5 ml) and the reaction mixture was kept under stirring at room temperature for 1.5 h until total disappearance of the reagent controlled by TLC. Subsequently the solution was neutralised with an aqueous solution of sodium bicarbonate 10% and vacuum evaporated. The residue was purified using a chromatographic column (chloroform/methanol 9:1) to give the compound of formula (I) α or β as white solid.

### EXAMPLE 11

### Preparation of the compound of formula (I) wherein R₂ is Et, R₁ is Me and B is cytosine

Starting from the epimeric mixture of isoxazolidine of formula (V) and (VI) prepared as described above in Example 3, and proceeding as described above in Example 4 replacing the fluorouracil by cytosine, the compound of the title was prepared.

### EXAMPLE 12

### Preparation of the compound of formula (I) wherein R₂ is Et, R₁ is Me and B is thymine

Starting from the epimeric mixture of isoxazolidine of formula (V) and (VI) prepared as described above in Example 3, and proceeding as described above in Example 4 replacing fluorouracil by thymine, the compound of the title was prepared.

### EVALUATION OF ANTIVIRAL ACTIVITY

The action of the present nucleoside analogues of general formula (I) on the infection *in vitro* of human retroviruses and human herpes viruses was evaluated.

To assay the antiretroviral activity, the infections *in vitro* of HTLV-1 (human T lymphotropic retrovirus type 1) and of HIV-1 (human immunodeficiency virus 1) were used. The capacity of these compounds to inhibit the activity of a retroviral reverse transcriptase *in vitro* was also assayed. To assay the anti-herpes virus activity *in vitro* infections of HSV-1 (*herpes simplex type 1*) and of HSV-2 (*herpes simplex type 2*) were used.

By way of example, all the assays for evaluation of the antiviral activity were performed on the following three compounds of formula (I), identified hereunder for simplicity as compounds A, B and C:
Compound A: compound of formula (I) wherein R₁= Me; R₂= Et; B= Cytosine, prepared as described in Example 11;
Compound B: compound of formula (I) wherein R₁= Me; R₂= Et; B= Thymine, prepared as described in Example 12;
Compound C: compound of formula (I) wherein R₁= Me; R₂= Et; B= fluorouracil, prepared as described in Example 4.

For all three compounds, cytotoxicity assays were performed at 72 h, evaluating the percentage of total dead cells, by trypan blue exclusion tests, or the percentage of apoptotic cells, by microscopy evaluation after staining with acridine orange, in a lymphoid cell line (Molt-3) and in a monocytoid cell line (U937), in concentrations of the compounds ranging from 1 µM to 400 µM, and calculating the CC₅₀ (concentration capable of causing total mortality evaluated with trypan blue in 50% of the cells) and AC₅₀ (concentration capable of causing apoptosis evaluated by staining with acridine orange in 50% of the cells). For all three compounds both CC₅₀ and AC₅₀ were > 400 µM.

HTLV-1 was the first human retrovirus identified as etiological agent of malignant neoplasias and progressive forms of neurological pathologies. This virus preferentially infects the CD4+ lymphocytes of humans, integrating into the host cell genome.

To assay the capacity to inhibit transmission, lymphomonocytes of adult peripheral blood were separated by density gradient centrifugation and then infected with HTLV-1 by co-cultivation with a chronically infected line that produces viruses, following irradiation of the donor cells (Miyoshi et al. Nature 1981, 296: 770-772).

The assayed nucleoside analogues (compounds A, B and C) were added at different concentrations at the moment of infection and the treatment was repeated at 3, 7 and 10 days from the first exposure.

The infection was monitored by verifying that the cells treated had become infected, compared to untreated controls, four weeks after infection. The infection was determined by monitoring the presence of proviral DNA and the viral RNA expression, by DNA-PCR and RT-PCR assay respectively. With these techniques it is possible to amplify and highlight the viral genome of HTLV-1 integrated into the host cell, that is virus-specific RNA-messengers, by the use of specific oligonucleotides for viral regions.

For further evaluation of the infection the viral protein expression of HTLV-1 Tax was also highlighted using Western blot analysis with specific monoclonal antibodies.

Moreover, the infected and uninfected cultures, treated or untreated with the compounds in question, were assayed to determine the proliferative response during infection, in order to verify, in addition to the antiviral effect also any antiproliferative effect with regard to the infected cultures. The results highlighted to the following results.

All three compounds assayed effectively protected the lymphomonocytes from HTLV-1 infection *in vitro,* in concentrations ranging from 100 µM to 1 µM. Besides being protective, the 100 µM or 50 µM concentrations were also partially antiproliferative both towards infected and uninfected cells. Even at the lowest concentrations (5µM and 1 µM) the compound C still offered total protection from the infection, exhibiting poor or no antiproliferative action. Treatments initiated subsequent to infection had no protective effect. The protective antiretroviral activity of these molecules can be compared to the activity obtained with the nucleoside analogue zidovudine (AZT), evaluated with the same methods (Macchi et al. J. Gen. Virol. 1997, 78: 1007-1016).

The HIV-1 retrovirus is the etiological agent of acquired immunodeficiency syndrome (AIDS). Over the years several viral strains have been isolated directly from patients and have been defined according to their tropism for T cells (T-tropic) or for cells of monocyte-macrophage origin (M-tropic). The strain HIV-1 mixed with HIV-III_{B} produced in chronically infected cells of lymphoid origin H-9 and therefore capable of growing the virus stably in a culture, was used to assay the anti-HIV activity. Viral inoculation was prepared by low speed centrifugation (3000 rpm) of the supernatant from the line producing the virus and by subsequent ultracentrifugation (40,000 rpm). The concentrated virus was titrated in TCID 50%/ml (units infecting 50% of the cultures). Cells from the lymphoid T-cell line, CEM, were used as cells to infect. The target cells were placed in contact with various quantities of TCID 50% of titrated virus, in an interval ranging from a MOl (multiplicity of infection) of 0.0001 and 0.1 TCID 50%/cell. For infection, microcultures of 2x10⁵ cells/200 microliters of culture medium were set up, which were exposed to viral inoculation in the presence of AZT concentrations ranging from 0.004 to 0.4 µM, as positive reference control, or of the compounds A, B and

C, ranging from 0.004 to 400 µM. The cultures were kept for 7 days at 37°C in the presence of fetal bovine serum 2%. After 2-4 days of culture the medium was removed and replaced with fresh medium to which the compounds in question were again added.

After seven days of setting up the culture, the infection was evaluated using the ELISA method, measuring the production of HIV-1 p24 protein in the supernatant of infected cultures, treated and untreated. The results of these assays showed that, at concentrations of 0.004 and 0.04 µM, AZT caused inhibition of the production of p24 in the supernatant of infected cultures of 60% and of 80% respectively. At the concentration of 0.4 µM, AZT guarantees an inhibition of 98%.

The compounds A, B and C did not cause inhibition at the concentration of 0.004 µM, while they caused inhibition of 45%, 50% and 60% respectively at the concentration of 0.04 µM, and inhibition of 70%, 70% and 75% respectively at the concentration of 0.4 µM. Finally, all three compounds caused a protection of 90% at the concentration of 4 µM and of 98% at the concentration of 40 µM. At higher concentrations inhibition was complete.

The compounds in question therefore proved to be capable of protecting susceptible cells from infection with HIV *in vitro,* at non-toxic concentrations.

In addition to the tests on cells susceptible to infection by human retroviruses, an assay was set up to highlight the capacity of the compounds A, B and C to inhibit *in vitro* the activity of a retroviral reverse transcriptase (RT). In short, the assay is based on the principle that starting from an RNA template, it is possible to evaluate the effect of inhibition of the capacity of the RT to synthesise cDNA, by identifying the cDNA fragment of neosynthesis, following gel electrophoretic run, thanks to incorporation of a nucleotide precursor labelled with phosphorus 32 (P₃₂). The absence of the fragment or the presence of labelled retrotranscriptase products with a lower molecular weight than expected, prove total or partial inhibition of the RT enzyme. As in order to be utilisable by the RT, the nucleoside analogues must be transformed into their triphosphate form, the compounds in question were placed in contact with cell protein extracts containing kinases capable of inducing phosphorylations. For this purpose protein extracts were used, obtained by lysis from a million PBMC previously stimulated in 48h of culture with PHA. The nucleoside compounds were incubated in ice for one hour with the lysates. After incubation the samples were taken to 95°C in order to inactivate the cellular enzymes present in the lysates. The activated nucleoside compounds (cell extract preincubated with the nucleoside analogue) were then incubated for 1 h at 37°C, at concentrations of 0.1, 1 and 20 µM with a mixture containing the following reagents: a commercially available RT of avian retrovirus (50U), total cytoplasmatic RNA extracted from the CEM cell line chronically infected by HIV (RNA template, 1 mg), a specific antisense primer for the HIV Tat/Rev splicing region, a pool of nucleotides (dNTP), a nucleotide labelled with phosphorus 32 in the alpha position (α³²P-ATP, 25 µCi). Subsequently the samples thus prepared were incubated at 95°C for 5 minutes, then loaded on an acrylamide gel 12% and subjected to electrophoretic run for approximately two hours at 200 volts. The molecular weights of the DNA fragments obtained, visualised after drying of the gel and autoradiography, were compared to those of a marker with known molecular weights. The assay was set up in the same way also for AZT, as positive control nucleoside compound, except for the concentrations used, which were 0.1 and 10 µM. The results of this assay proved that both AZT and the compounds A, B and C at all the concentrations utilised were capable of inhibiting RT activity.

To assay the activity of the compounds also towards herpes virus infections, the model for *in vitro* transmission of *herpes simplex type 1* (HSV-1) and of *herpes simplex type 2* (HSV-2) to permissive cells of the Vero cell line and plaque reduction assay were used. Vero cells were transferred and cultivated for 24h at 37°C in six-well plates for the cultures to reach confluence. After removal of the culture medium in the wells, a suspension was added containing 100 plaque forming units (PFU) of HSV-1, strain "F", or HSV-2, strain "G" in 100 µl of medium. After one hour of incubation the appropriate quantities of compounds were added diluted in culture medium with the addition of serum 2% and methylcellulose 0.8% to obtain final concentrations of the compounds ranging from 1 µM to 400 µM. The cultures were then incubated for 72h, the medium was removed from the wells and stained with crystal violet 0.8%. After removing the wash liquid, the plates were left to dry and the number of plaques was determined. Acyclovir was used as the positive reference control. A double assay was performed and the values of EC₅₀ (concentration capable of reducing the formation of plaques by 50%) were calculated by extrapolating the mean number of plaques from the plot towards the corresponding concentrations of the compounds.

The results obtained are indicated in Table 1 below.

**Table 1**

| | EC₅₀ (µM) compound A | EC₅₀ (µM) compound B | EC₅₀ (µM) compound C | EC₅₀ (µM) acyclovir |
|---|---|---|---|---|
| HSV-1 | 48 | 62 | 34 | 14 |
| HSV-2 | 72 | 78 | 56 | 32 |

Therefore, also in this case the assayed compounds proved to have a certain degree of protective activity against the infection at non-toxic doses.

The properties shown in the tests for cytotoxicity, protection of viral infection *in vitro* and inhibition of the retroviral reverse transcriptase activity *in vitro,* thus indicate the efficacy of the present nucleoside analogues of formula (I) as antiviral agents.

## Claims

1. Nucleoside analogues of general formula (I) 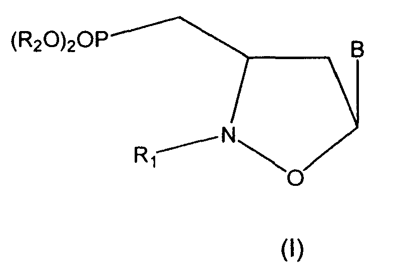 wherein:
B is a group **selected from: thymine, n-acetvlcytosine, uracil, 5-fluorouracil, cytosine, guanine, adenine**,
R₁ is selected from the group consisting of H, C1-C4 alkyl groups, aryl groups, and glycoside units,
R₂ is selected from the group consisting of H, C1-C4 alkyl groups, aryl groups, and **protective groups easily hydrolyzable *in vivo*** and their pharmaceutically acceptable salts.

2. Nucleoside analogues of general formula (I) according to claim 1, wherein said group B is selected from the group consisting of thymine, cytosine and fluorouracil.

3. Nucleoside analogues of general formula (I) according to claim 1, wherein said glycoside unit is ribosyl acetonide.

4. Nucleoside analogues of general formula (I) according to claim 1, wherein said aryl groups are selected from benzyl groups, **pyridyl and pyrrole groups**.

5. Nucleoside analogues of general formula (I) according to claim 1, wherein R₁ is selected from between H and methyl.

6. Nucleoside analogues of general formula (I) according to claim 1, wherein R₂ is ethyl.

7. Nucleoside analogues of general formula (I) according to claim 1, wherein said **protective groups easily hydrolyzable *in vivo*** are selected from the isopropyloxymethylcarbonyl (POC) group and the pivaloyloxymethyl (POM) group.

8. Process for the preparation of the nucleoside analogues of formula (I) as described in claims 1-7, comprising the following steps:
i) hydrolysis of the diethyl acetal of phosphonoacetaldehyde of formula (II) to obtain the phosphonoacetaldehyde of formula (III) 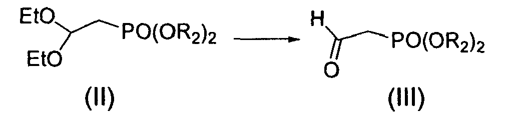 wherein R₂ is as defined in claim 1;
ii) reaction of the diethylphosphonoacetaldehyde of formula (III) coming from step i) with an alkyl hydroxylamine of formula R₁NHOH to obtain the nitrone of formula (IV) 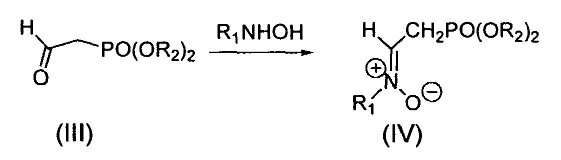 wherein R₁ and R₂ are as defined in claim 1;
iii) cycloaddition of the nitrone of formula (IV) coming from step ii) with vinylacetate to obtain the mixture of isoxazolidine epimers of formula (V) and (VI) 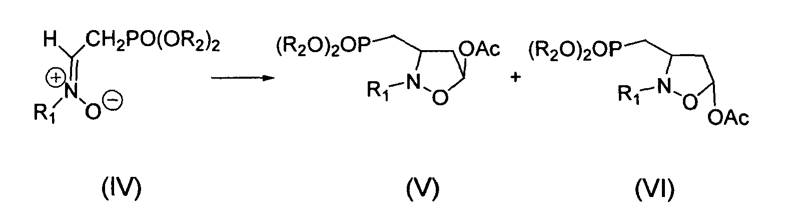 wherein R₁ and R₂ are defined as above;
iv) reaction of the epimeric mixture of the compounds of formula (V) and (VI) obtained from step iii) with a silylated nucleic acid base or a derivative thereof, to obtain the nucleoside analogue of formula (I) 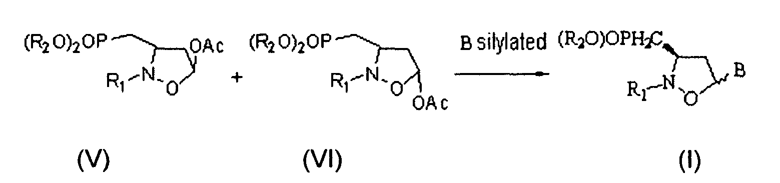
wherein R₁ and R₂ are defined as above, B is a nucleic acid base, and the bond represented by the broken line indicates that the compound of formula (I) is obtained in the form of mixtures of α- and β- nucleosides.

9. Process according to claim 8, wherein said step i) is carried out by treatment of the diacetal of formula (II) with an ion exchange resin in acetone at room temperature.

10. Process according to claim 8, wherein said step ii) is carried out in dichloromethane.

11. Process according to claim 8, wherein in said step iii) an epimeric mixture is obtained in which the ratio between the epimer of formula (V) and the epimer of formula (VI) is 2.5:1.

12. Process according to claim 8, wherein the crude mixture of the two epimers of formula (V) and (VI) obtained from said step iii) is purified to obtain the two separated epimers, which are then subjected separately to the subsequent reaction step iv) with the silylated bases.

13. Process according to claim 8, wherein said step iv) is carried out in acetonitrile at a temperature ranging from 20 to 70°C, in the presence of a quantity of trimethylsilyl trifluoromethanesulfonate ranging from 0.1 to 0.4 equivalents, as catalyst.

14. Process according to claim 8, wherein said step iv) is carried out in an anhydrous organic solvent selected from the group consisting of acetonitrile, dichloromethane, dichloroethane, tetrahydrofurane and dimethylformamide, at a temperature ranging from 20 to 70°C in the presence of a quantity of stannic chloride ranging from 0.1 to 0.4 equivalents, as catalyst.

15. Process for the preparation of the nucleoside analogues of formula (I) as described in claims 7, comprising the reaction of cycloaddition of the nitrone of formula (IV) with vinyl nucleobases to obtain the mixture of epimeric nucleosides of formula (I): 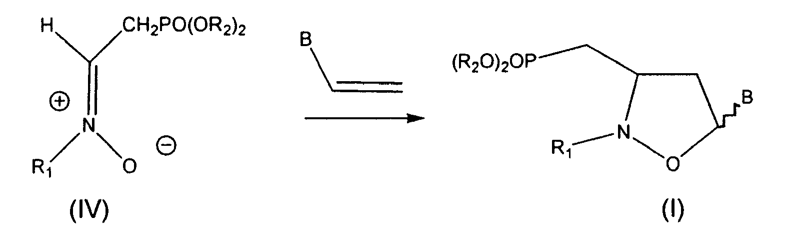 wherein R₁ and R₂ are as defined in claim 1.

16. Process according to claim 15, wherein said cycloaddition reaction is carried out in toluene at a temperature ranging from 80 to 90°C for 24 hours.

17. Process for the preparation of the nucleoside analogues of formula (I) as described in claims 17, comprising the following steps:
i') cycloaddition of the nitrone of formula (VII) with vinylacetate to obtain the mixture of isoxazolidine epimers of formula (VIII) and (IX) 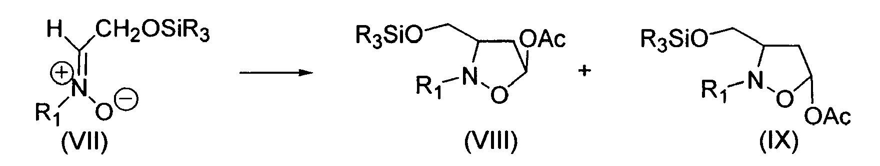 wherein R₁ is defined as in claim 1, and R₃ is a C1-C4 alkyl group;
ii') reaction of the epimeric mixture of the compounds of formula (VIII) and (IX) obtained from step i') with a silylated nucleic acid base, wherein R₁ and R₃ are defined as above, B is a nucleic acid base, and the bond represented by the broken line indicates that the compound of formula (X) is obtained in the form of mixture of α- and β- nucleosides: 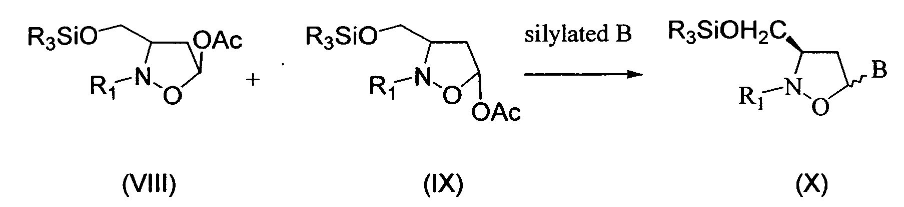 wherein B, R₁ and R₃ are defined as above;
iii') reaction of the epimeric mixture of the compound of formula (X), obtained from step ii'), with TBAF and subsequently thionyl chloride: 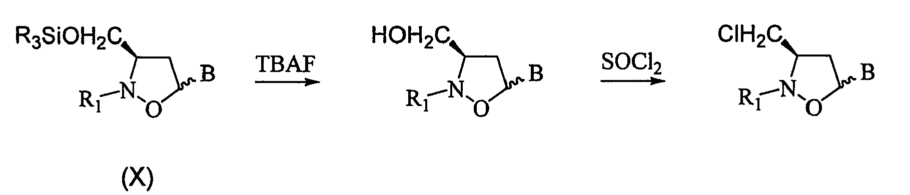 wherein B, R₁ and R₃ are as defined above;
iv') Arbuzov reaction with triethyl phosphite to obtain the α- and β-nucleosides of formula (I) as defined in claim 1, wherein R₂ is Ethyl:

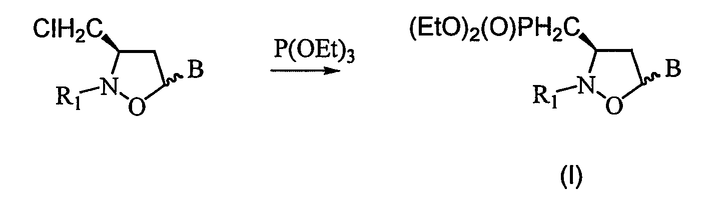

18. Process for the preparation of the nucleoside analogues of formula (I) as described in claims 1-7, wherein R₁ is constituted by H or a glycoside unit and R₂ is ethyl in enantiomerically pure form, comprising the following steps:
i") reaction of vinylacetate and diethylphosphonoaldehyde with **an appropriate glycoside of the formula (XIII)** 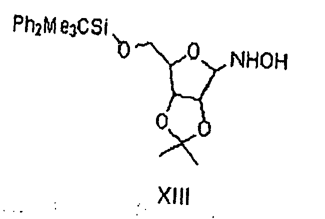 to obtain a mixture of the two corresponding epimeric isoxazolidines in enantiomerically pure form;
ii") nucleosidation of the epimeric mixture obtained from step i") to obtain the nucleosides of formula (I) wherein R₁ is a glycoside unit in the form of epimeric mixture;
iii") chromatographic separation of the epimeric mixture coming from step ii") to obtain the two nucleosides of formula (I) in enantiomerically pure form, wherein R₁ is a protected glycoside unit;
iv") deprotection of the glycoside unit of the two nucleosides coming from step iii") to obtain the two nucleosides of formula (I) in enantiomerically pure form, wherein R₁ is a glycoside unit;
v") optional elimination of the glycoside unit from the two nucleosides obtained from stage iv") to obtain the two nucleosides of formula (I), wherein R₁ is H in enantiomerically pure form.

19. Pharmaceutical compositions comprising as active ingredient at least one of the nucleoside analogues of general formula (I) as described in claims 1-7, optionally in combination with other active ingredients or adjuvants.

20. Pharmaceutical compositions according to claim 19, further comprising pharmaceutically acceptable excipients and/or diluents.

21. Use of the nucleoside analogues of general formula (I) as defined in claims 17 for the preparation of pharmaceutical compositions useful for the treatment of viral pathologies.

22. Use of the nucleoside analogues of general formula (I) according to claim 21, wherein said viral pathologies are selected from the group consisting of retroviruses, hepatic viruses and herpes viruses.

## Patentansprüche

1. Nukleosidanaloga mit allgemeiner Formel (I) 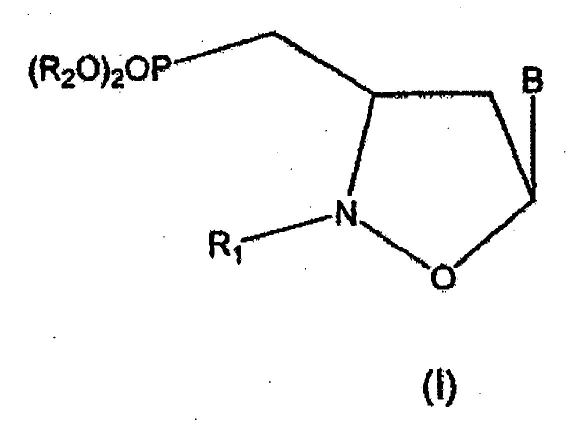 wobei:
B eine Gruppe ist, **die ausgewählt ist unter: Thymin, n-Acetylcytosin, Uracil, 5-Fluorouracil, Cytosin, Guanin, Adenin,**
R₁ aus der Gruppe ausgewählt ist, die aus H, C1-C4 Alkylgruppen, Arylgruppen und Glykosideinheiten besteht,
R₂ ausgewählt ist aus der Gruppe, die aus H, C1-C4 Alkylgruppen, Arylgruppen und aus ***in vivo* leicht hydrolisierbaren Schutzgruppen** und deren pharmazeutisch anerkannten Salzen besteht.

2. Nukleosidanaloga mit allgemeiner Formel (I) gemäß Anspruch 1, wobei genannte Gruppe B ausgewählt ist aus der Gruppe, welche aus Thymin, Cytosin und Fluorouracil besteht.

3. Nukleosidanaloga mit allgemeiner Formel (I) gemäß Anspruch 1, wobei es sich bei der genannten Glykosideinheit um Ribosylacetonid handelt.

4. Nukleosidanaloga mit allgemeiner Formel (I) gemäß Anspruch 1, wobei genannte Arylgruppen ausgewählt sind unter Benzylgruppen, **Pyridyl- und Pyrrolgruppen.**

5. Nukleosidanaloga mit allgemeiner Formel (I) gemäß Anspruch 1, wobei R₁ ausgewählt ist unter H und Methyl.

6. Nukleosidanaloga mit allgemeiner Formel (I) gemäß Anspruch 1, wobei es sich bei R₂ um Ethyl handelt.

7. Nukleosidanaloga mit allgemeiner Formel (I) gemäß Anspruch 1, wobei die genannten ***in vivo* leicht hydrolysierbaren Schutzgruppen** ausgewählt sind aus der Isopropyloxymethylcarbonyl (POC)-Gruppe und der Pivaloyloxymethyl (POM)-Gruppe.

8. Verfahren für die Herstellung der Nukleosidanaloga mit Formel (I), wie in den Ansprüchen 1-7 beschrieben, welches folgende Schritte umfasst:
i) Hydrolyse des Diethylacetals von Phosphonoacetaldehyd mit Formel (II), um das Phosphonoacetaldehyd mit Formel (III) zu erhalten, 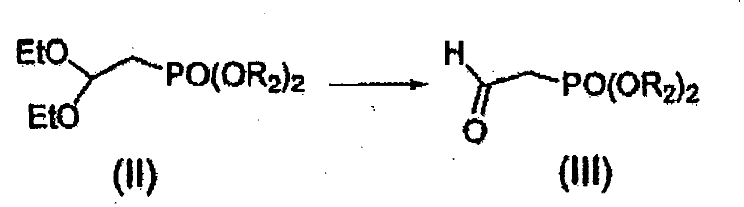 wobei R₂ wie in Anspruch 1 definiert ist;
ii) Zur Reaktion bringen des Diethylphosphonoacetalaldehyds mit Formel (III), welches aus Schritt i) stammt, mit einem Alkylhydroxylamin mit Formel R₁NHOH, um das Nitron mit Formel (IV) zu erhalten, 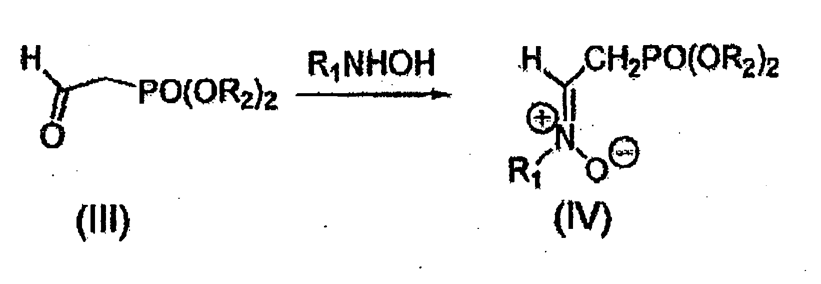 wobei R₁ und R₂ wie in Anspruch 1 definiert sind;
iii) Cycloaddition des Nitron mit Formel (IV), welches aus Schritt ii) stammt mit, Vinylacetat, um die Mischung von Isoxazolidinepimeren mit Formel (V) und (VI) zu erhalten, 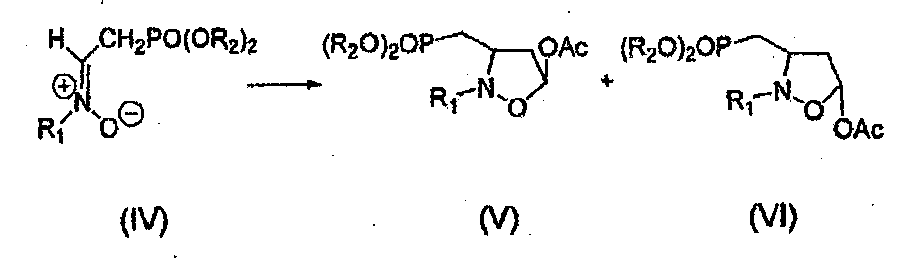 wobei R₁ und R₂ wie oben definiert sind;
iv) Zur Reaktion bringen der Epimermischung aus den Verbindungen mit Formel (V) und (VI), die in Schritt iii) erhalten werden, mit einer silierten Nukleinsäurebase oder einem Derivat davon, um das Nukleosidanalog mit Formel (I) zu erhalten, 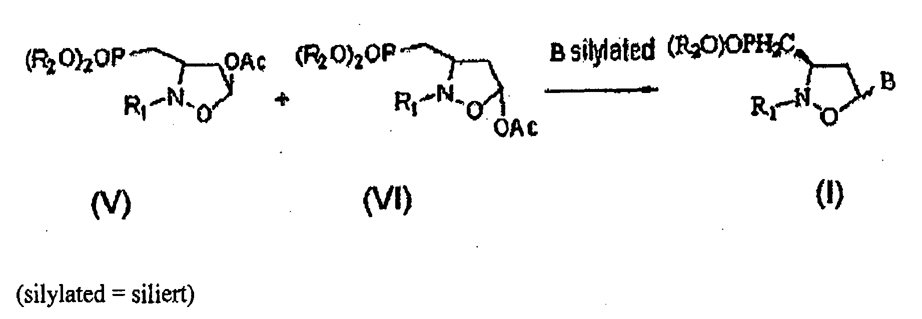
wobei R₁ und R₂ wie oben 1 definiert sind, B eine Nukleinsäurebase ist und die Bindung, die durch die unterbrochene Linie dargestellt ist, bedeutet, dass die Verbindung mit Formel (I) in der Form von Mischungen aus α- und β-Nukleosiden erhalten wird.

9. Verfahren gemäß Anspruch 8, wobei genannter Schritt i) durchgeführt wird, indem das Diacetal mit Formel (II) mit einem Ionenaustauscherharz in Aceton bei Raumtemperatur behandelt wird.

10. Verfahren gemäß Anspruch 8, wobei genannter Schritt ii) in Dichlormethan durchgeführt wird.

11. Verfahren gemäß Anspruch 8, wobei in genanntem Schritt iii) eine Epimermischung erhalten wird, in welcher das Verhältnis zwischen dem Epimer mit Formel (V) und dem Epimer mit Formel (VI) 2,5:1 beträgt.

12. Verfahren gemäß Anspruch 8, wobei die Rohmischung der beiden Epimere mit Formel (V) und (VI), welche aus genanntem Schritt iii) erhalten wird, gereinigt wird, um die beiden Epimere getrennt zu gewinnen, welche dann einzeln dem nachfolgenden Reaktionsschritt iv) mit den silierten Basen unterzogen werden.

13. Verfahren gemäß Anspruch 8, wobei genannter Schritt iv) in Acetonitril bei einer Temperatur im Bereich von 20 °C bis 70 °C in Anwesenheit einer Menge von Trimethylsilyltrifluormethansulfonat im Bereich von 0,1 bis 0,4 Äquivalenten als Katalysator durchgeführt wird.

14. Verfahren gemäß Anspruch 8, wobei genannter Schritt iv) in einem wasserfreien organischen Lösungsmittel, welches ausgewählt ist aus der Gruppe, die aus Acetonitril, Dichlormethan, Dichlorethan, Tetrahydrofuran und Dimethylformamid besteht, bei einer Temperatur im Bereich von 20 °C bis 70°C in Anwesenheit einer Menge von Zinnchlorid im Bereich von 0,1 bis 0,4 Äquivalenten als Katalysator durchgeführt wird.

15. Verfahren für die Herstellung der Nukleosidanaloga mit Formel (I), wie in Ansprüchen 1-7 beschrieben, welches die Reaktion der Cycloaddition des Nitrons mit Formel (IV) mit Vinylnukleobasen umfasst, um die Mischung von epimeren Nukleosiden mit Formel (I) zu erhalten: 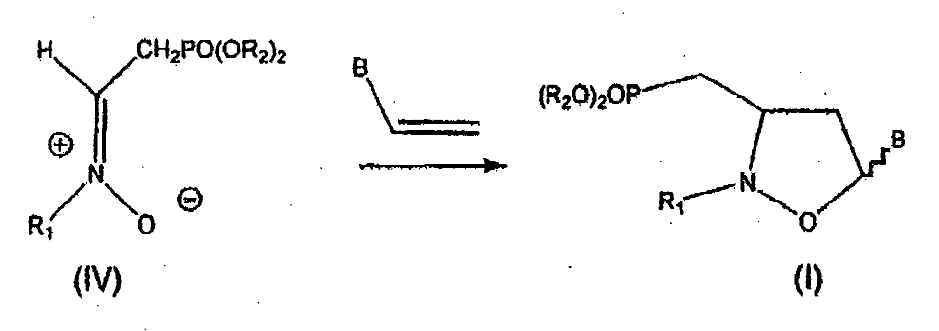 wobei R₁ und R₂ wie in Anspruch 1 definiert sind.

16. Verfahren gemäß Anspruch 15, wobei genannte Cycloadditionsreaktion in Toluol bei einer Temperatur im Bereich von 80 °C bis 90 °C für 24 Stunden durchgeführt wird.

17. Verfahren für die Herstellung der Nukleosidanaloga mit Formel (I), wie in Ansprüchen 1-7 beschrieben, welches die folgenden Schritte umfasst:
i') Cycloaddition des Nitrons mit Formel (VII) mit Vinylacetat, um die Mischung von Isoxazolidinepimeren mit Formel (VIII) und (IX) zu erhalten, 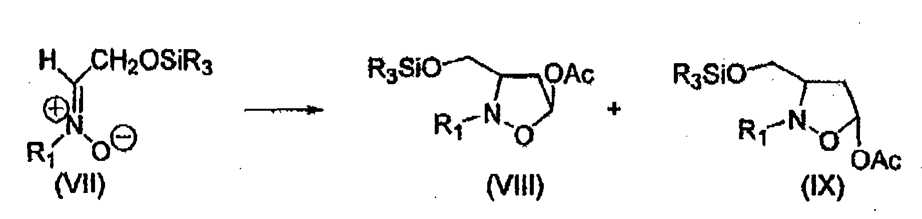 wobei R₁ wie in Anspruch 1 definiert ist und es sich bei R₃ um eine C1-C4 Alkylgruppe handelt;
ii') Reaktion der Epimermischung der Verbindungen mit Formel (VIII) und (IX), die in Schritt i') erhalten wird, mit einer silierten Nukleinsäurebase, wobei R₁ und R₃ wie oben definiert sind, B eine Nukleinsäurebase ist und die Bindung, die durch die unterbrochene Linie dargestellt ist, bedeutet, dass die Verbindung mit Formel (X) in der Form einer Mischung von α- und β-Nukleosiden erhalten wird: 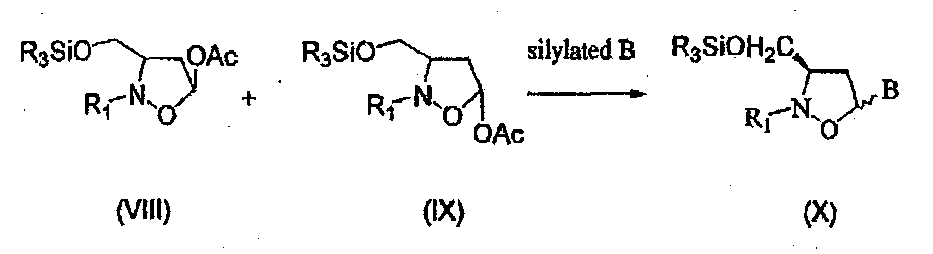 wobei B, R₁ und R₃ wie oben definiert sind;
iii') Reaktion der Epimermischung der Verbindungen mit Formel (X), welche in Schritt ii') erhalten wird, mit TBAF und nachfolgend mit Thionylchlorid: 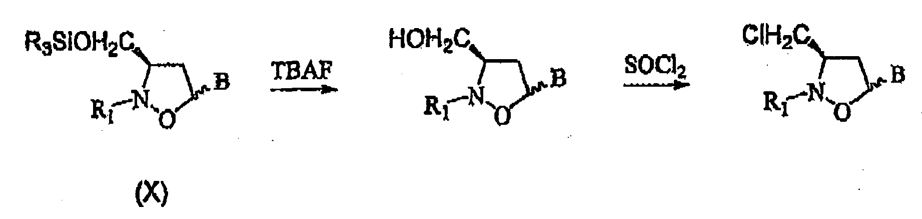 wobei B, R₁ und R₃ wie oben definiert sind;
iv') Arbuzov-Rektion mit Triethylphosphit, um die α- und β-Nukleoside mit Formel (I) zu erhalten, wie in Anspruch 1 definiert, wobei es sich bei R₂ um Ethyl handelt:

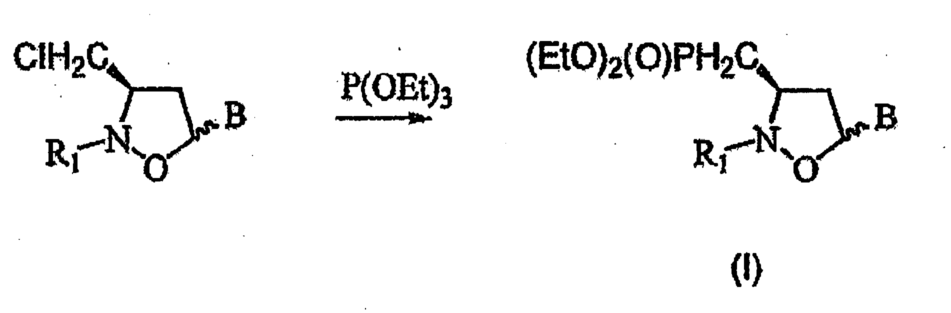

18. Verfahren für die Herstellung der Nukleosidanaloga mit Formel (I), wie in Ansprüchen 1-7 beschrieben, wobei R₁ durch H oder eine Glykosideinheit gebildet ist und R₂ Ethyl in enantiomer reiner Form ist, welches die folgenden Schritte umfasst:
i") Reaktion von Vinylacetat und Diethylphophonoaldehyd mit einem **geeigneten Glykosid mit der Formel (XIII),** 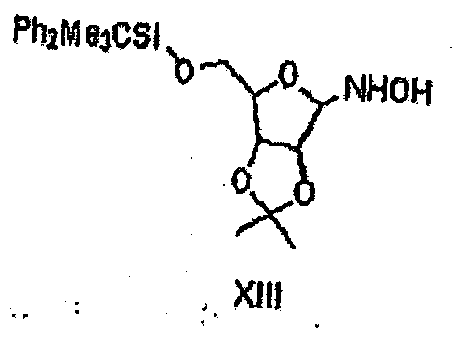 um eine Mischung aus zwei entsprechenden epimeren Isoxazolidinen in enationmer reiner Form zu erhalten;
ii") Nukleosierung der Epimermischung, welche in Schritt i") erhalten wird, um die Nukleoside mit Formel (I) zu erhalten, wobei es sich bei R₁ um eine Glykosideinheit in der Form einer Epimermischung handelt;
iii") Chromatografische Trennung der Epimermischung, welche in Schritt ii") erhalten wird, um die beiden Nukleoside mit Formel (I) in enantiomer reiner Form zu erhalten, wobei es sich bei R₁ um eine geschützte Glykosideinheit handelt;
iv") Entschützen der Glykosideinheit der beiden Nukleoside, die in Schritt iii") erhalten werden, um die beiden Nukleoside mit Formel (I) in enantiomer reiner Form zu erhalten, wobei es sich bei R₁ um eine Glykosideinheit handelt;
v") Optionales Entfernen der Glykosideinheit von den beiden Nukleosiden, die in Schritt iv") erhalten werden, um die beiden Nukleoside mit Formel (I) zu erhalten, wobei es sich bei R₁ um H in enantiomer reiner Form handelt.

19. Pharmazeutische Zusammensetzungen, welche als aktiven Inhaltstoff mindestens eines der Nukleosidanaloga mit allgemeiner Formel (I), wie in Ansprüchen 1-7 beschrieben, optional in Kombination mit weiteren aktiven Inhaltsstoffen oder Adjuvantien umfassen.

20. Pharmazeutische Zusammensetzungen gemäß Anspruch 19, welche ferner pharmazeutisch anerkannte Hilfsstoffe und/oder Verdünnungsmittel umfassen.

21. Verwendung der Nukleosidanaloga mit allgemeiner Formel (I), wie in Ansprüchen 1-7 definiert, für die Herstellung von pharmazeutischen Zusammensetzungen, welche hilfreich für die Behandlung von viralen Erkrankungen sind.

22. Verwendung der Nukleosidanaloga mit allgemeiner Formel (I) gemäß Anspruch 21, wobei genannte virale Erkrankungen ausgewählt sind aus der Gruppe, die durch Retroviren, Hepatoviren und Herpesviren verursacht werden.

## Revendications

1. Analogues de nucléosides de formule générale (I) 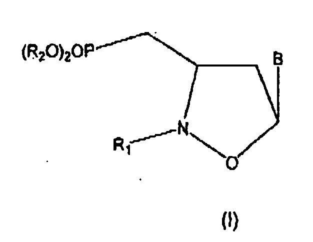 dans lesquels :
B est un groupe choisi parmi :la thymine, la n-acétylcytosine, l'uracile, le 5-fluorouracile, la cytosine, la guanine, l'adénine,
R₁ est choisi dans le groupe constitué de H, de groupes alkyle en C₁-C₄, de groupes aryle, et de motifs glycosidiques,
R₂ est choisi dans le groupe constitué de H, de groupes alkyle en C₁-C₄, de groupes aryle et de groupes protecteurs facilement hydrolysables in vivo et de leurs sels pharmaceutiquement acceptables.

2. Analogues de nucléosides de formule générale (I) selon la revendication 1, dans lesquels ledit groupe B est choisi dans le groupe constitué de la thymine, de la cytosine et du fluorouracile.

3. Analogues de nucléosides de formule générale (I) selon la revendication 1, dans lesquels ledit motif glycosidique est du ribosyle acétonide.

4. Analogues de nucléosides de formule générale (I) selon la revendication 1, dans lesquels lesdits groupes aryle sont choisis parmi des groupes benzyle, des groupes pyridyle et pyrrole.

5. Analogues de nucléosides de formule générale (I) selon la revendication 1, dans lesquels R₁ est choisi entre H et le groupe méthyle.

6. Analogues de nucléosides de formule générale (I) selon la revendication 1, dans lesquels R₂ est le groupe éthyle.

7. Analogues de nucléosides de formule générale (I)selon la revendication 1, dans lesquels lesdits groupes protecteurs facilement hydrolysables in vivo sont choisis parmi le groupe isopropyloxyméthyl-carbonyle (POC) et le groupe pivaloyloxyméthyle (POM).

8. Procédé de préparation des analogues de nucléosides de formule (I) tels que décrits dans les revendications 1 à 7, comprenant les étapes suivantes :
i) l'hydrolyse de phosphonoacétaldéhyde diéthyle acétal de formule (II) pour obtenir le phosphonoacétaldéhyde de formule (III) 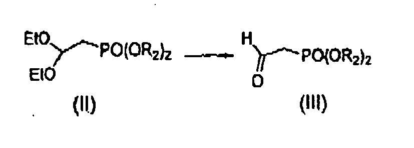 dans lequel R₂ est tel que défini dans la revendication 1;
ii) la réaction du diéthylphosphonoacétaldéhyde de formule (III) provenant de l'étape i) avec une alkylhydroxylamine de formule R₁-NHOH pour obtenir la nitrone de formule (IV) 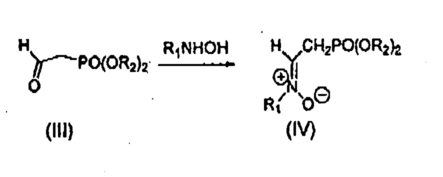 dans laquelle R₁ et R₂ sont tels que définis dans la revendication 1;
iii) la cycloaddition de la nitrone de formule (IV) provenant de l'étape ii) avec de l'acétate de vinyle pour obtenir le mélange d'épimères d'isoxazolidine de formule (V) et (VI) 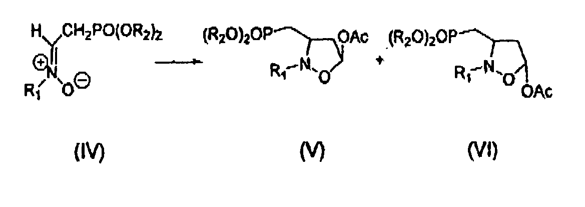 dans lequel R₁ et R₂ sont tels que définis ci-dessus ;
iv) la réaction du mélange épimérique des composés de formule (V) et (VI) obtenus à l'étape iii) avec une base d'acide nucléique silylée ou son dérivé, pour obtenir l'analogue de nucléoside de formule (I) 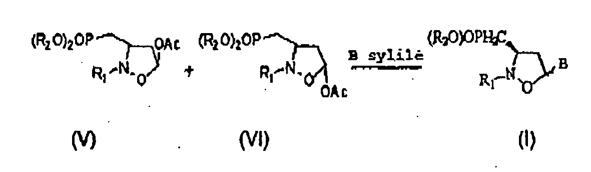
dans lequel R₁ et R₂ sont tels que définis ci-dessus, B est une base d'acide nucléique, et la liaison représentée par la ligne discontinue indique que le composé de formule (I) est obtenu sous la forme de mélanges de α- et β-nucléosides.

9. Procédé selon la revendication 8, dans lequel ladite étape i) est réalisée par traitement de l'acétal de formule (II) avec une résine échangeuse d'ions dans de l'acétone à température ambiante.

10. Procédé selon la revendication 8, dans lequel ladite étape ii) est réalisée dans du dichlorométhane.

11. Procédé selon la revendication 8, dans lequel dans ladite étape iii) un mélange épimérique est obtenu dans lequel le rapport entre l'épimère de formule (V) et l'épimère de formule (VI) est 2,5:1.

12. Procédé selon la revendication 8, dans lequel le mélange brut des deux épimères de formule (V) et (VI) obtenu à ladite étape iii) est purifié pour obtenir les deux épimères séparés, qui sont ensuite soumis séparément à l'étape de réaction suivante iv) avec des bases silylées.

13. Procédé selon la revendication 8, dans lequel ladite étape iv) est réalisée dans de l'acétonitrile à une température allant de 20 à 70 °C en présence d'une quantité de trifluorométhanesulfonate de triméthylsilyle allant de 0,1 à 0,4 équivalents, à titre de catalyseur.

14. Procédé selon la revendication 8, dans lequel ladite étape iv) est réalisée dans un solvant organique anhydre choisi dans le groupe constitué d'acétonitrile, de dichlorométhane, de dichloroéthane, de tétrahydrofurane et de diméthylformamide, à une température allant de 20 à 70 °C en présence d'une quantité de chlorure stannique allant de 0,1 à 0,4 équivalents, à titre de catalyseur.

15. Procédé de préparation d'analogues de nucléosides de formule (I) tels que décrits dans les revendications 1 à 7, comprenant la réaction de cycloaddition de la nitrone de formule (IV) avec des nucléobases vinyliques pour obtenir le mélange de nucléosides épimériques de formule (I) : 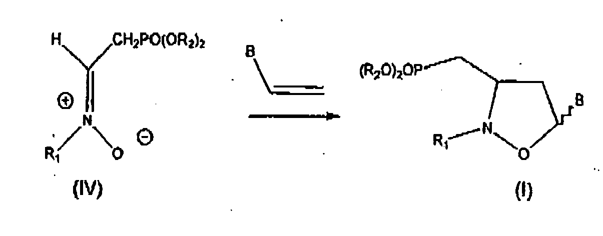 dans lequel R₁ et R₂ sont tels que définis dans la revendication 1.

16. Procédé selon la revendication 15, dans lequel ladite réaction de cycloaddition est réalisée dans du toluène à une température allant de 80 à 90°C pendant 24 heures.

17. Procédé de préparation d'analogues de nucléosides de formule (I) tels que décrits dans les revendications 1 à 7, comprenant les étapes suivantes :
i') la cycloaddition de la nitrone de formule (VII) avec de l'acétate de vinyle pour obtenir le mélange d'épimères d'isoxazolidine de formule (VIII) et (IX) 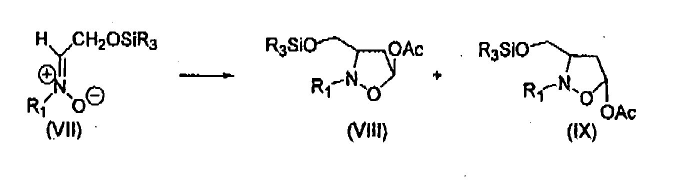 dans lequel R₁ est tel que défini dans la revendication 1, et R₃ est un groupe alkyle en C₁-C₄ ;
ii') la réaction du mélange épimérique des composés de formule (VIII) et (IX) obtenu à l'étape i') avec une base d'acide nucléique silylée, dans laquelle R₁ et R₃ sont tels que définis ci-dessus, B est une base d'acide nucléique, et la liaison représentée par la ligne discontinue indique que le composé de formule (X) est obtenu sous forme de mélange de α- et β-nucleosides : 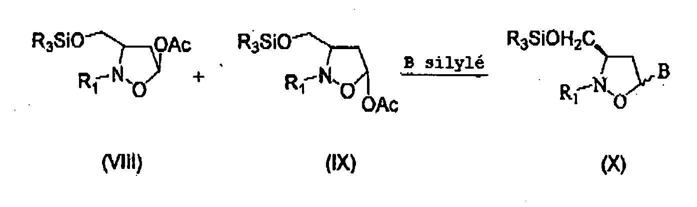 dans lequel B, R₁ et R₃ sont tels que définis ci-dessus ;
iii') la réaction du mélange épimérique du composé de formule (X), obtenu à l'étape ii'), avec TBAF et par la suite avec du chlorure de thionyle : 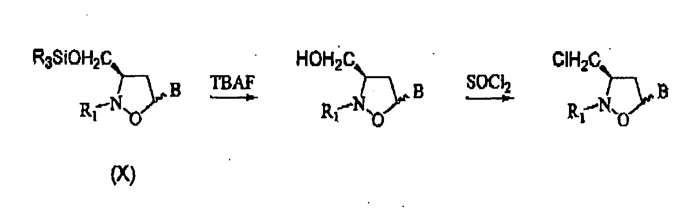 dans laquelle B, R₁ et R₃ sont tels que définis ci-dessus ;
iv') la réaction d'Arbuzov avec du phosphite de triéthyle pour obtenir les α- et β-nucléosides de formule (I) tels que définis dans la revendication 1, dans lesquels R₂ est le groupe éthyle :

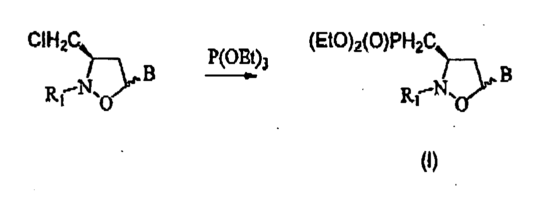

18. Procédé de préparation d'analogues de nucléosides de formule (I) tels que décrits dans les revendications 1 à 7, dans lequel R₁ est constitué par H ou un motif glycosidique et R₂ est le groupe éthyle sous forme énantiomériquement pure, comprenant les étapes suivantes :
i") la réaction de l'acétate de vinyle et du diéthylphosphonoaldéhyde avec un glycoside approprié de formule (XIII) 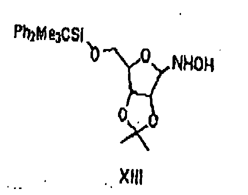 pour obtenir un mélange des deux isoxazolidines épimériques correspondantes sous forme énantiomériquement pure ;
ii") la nucléosidation du mélange épimérique obtenu à l'étape i") pour obtenir les nucléosides de formule (I) dans lesquels R₁ est un motif glycosidique sous la forme de mélange épimérique ;
iii") la séparation par chromatographie du mélange épimérique provenant de l'étape ii") pour obtenir les deux nucléosides de formule (I) sous forme énantiomériquement pure, dans lesquels R₁ est un motif glycosidique protégé ;
iv") la déprotection du motif glycosidique des deux nucléosides provenant de l'étape iii") pour obtenir les deux nucléosides de formule (I) sous forme énatiomériquement pure, dans lesquels R₁ est un motif glycosidique ;
v") l'élimination éventuelle du motif glycosidique des deux nucléosides obtenus à l'étape iv") pour obtenir les deux nucléosides de formule (I), dans lesquels R₁ est H sous forme énantiomériquement pure.

19. Compositions pharmaceutiques comprenant comme ingrédient actif au moins un des analogues de nucléosides de formule générale (I) tels que décrits dans les revendications 1 à 7, éventuellement en association avec d'autres ingrédients actifs ou adjuvants.

20. Compositions pharmaceutiques selon la revendication 19, comprenant en outre des excipients et/ou des diluants pharmaceutiquement acceptables.

21. Utilisation des analogues de nucléosides de formule générale (I) tels que définis dans les revendications 1 à 7 pour la préparation de compositions pharmaceutiques utiles pour le traitement de pathologies virales.

22. Utilisation des analogues de nucléosides de formule générale (I) selon la revendication 21, dans laquelle lesdites pathologies virales sont choisies dans le groupe constitué de rétrovirus, de virus hépatiques et de virus de l'herpès.
